Europäisches Patentamt

European Patent Office

Office européen des brevets

(19)

(11) Publication number: **0 101 398**
**B1**

(12) **EUROPEAN PATENT SPECIFICATION**

(45) Date of publication of patent specification : **18.06.86**

(51) Int. Cl.⁴ : **C 12 Q   1/06**

(21) Application number : **83630116.8**

(22) Date of filing : **15.07.83**

(54) **Method of concentrating and measuring unicellular organisms.**

(30) Priority : **21.07.82 US 400556**

(43) Date of publication of application :
**22.02.84 Bulletin 84/08**

(45) Publication of the grant of the patent :
**18.06.86 Bulletin 86/25**

(84) Designated contracting states :
**CH DE FR GB IT LI SE**

(56) References cited :
**FR-A- 2 172 041**
**US-A- 4 283 490**
**US-A- 4 336 337**
**GLOSSARY OF GENETICS AND CYTOGENETICS, p. 334 (1976)**
**NEW   PRACTICAL   DICTIONARY   FUNK   AND WAGNALLS; P: /)( Vol. 1**
**The file contains technical information submitted after the application was filed and not included in this specification**

(73) Proprietor : **Packard Instrument Company, Inc.**
**2200 Warrenville Road**
**Downers Grove, IL 60515 (US)**

(72) Inventor : **Tsai, Ten Lin Sie**
**7130 Summit Road**
**Darien Illinois 60559 (US)**

(74) Representative : **Schmitz, Jean-Marie et al**
**Office Dennemeyer S.à.r.l. 21-25 Allée Scheffer P.O. Box 41**
**L-2010 Luxembourg (LU)**

## Description

The present invention concerns a method of measuring the concentration of ATP-containing unicellular organisms in a sample, comprising the steps of concentrating the organisms by passing the sample through a filter membrane impermeable to the organisms, rupture the organisms to release ATP from said organisms, treating with a luminescent reagent to react with released ATP, thereby producing a luminescence directly on the filter membrane and measuring the luminescent response with a luminometer.

Quantitative determination of the presence of unicellular organisms (such as bacteria and yeast cells) is important in many areas. For example, the presence of bacteria in such things as paint, meat and water cooling towers is vital to the successful use of these products. If bacteria levels exceed a certain maximum, the economic damage can be devastating. Conventional methods useful for detecting the presence and/or amount of such cells in a particular area are generally time-consuming, relatively tedious and not very accurate when the cell concentration is less than about $1 \times 10^5$ cells per milliliter.

Accordingly, what is needed in this art is a simple method of detecting unicellular organisms even in relatively low concentrations.

The US-A-4 283 490 describes a method for detecting low concentrations of luminescent reactive molecules from microbial cells in samples of fluids and degraded solids is disclosed. A sample containing microbial cells is treated to eliminate substantially all nonmicrobial material. The microbial cells are then caused to rupture by physico-chemical means and form a thin film positioned in the vicinity of a photodetector. An appropriate reagent contacts the thin film and the resulting photon emission is observed by the photodetector. The strength of the luminescence is measured and recorded as an indication of the concentration of microbial cells in the sample.

The process of the present invention is characterized in that as said filter membrane a polycarbonate filter membrane is used and for rupturing the organisms, the filter membrane containing the concentrated organisms, is treated sequentially with a lysing agent.

The present invention is directed to a simple method of measuring unicellular organisms in samples including samples with low unicellular organism content. The method comprises concentrating the unicellular organism (cell) content of the sample by passing the sample through a polycarbonate filter membrane impermeable to the cells.

Once the cells are present on the polycarbonate filter membrane, the filter membrane is treated sequentially with a lysing agent and luminescent reagent. The lysing agent lyses the cell walls releasing the adenosine triphosphate (ATP) present in the cells. The ATP is then reacted with the luminescent reagent producing a luminescent response (light) which is measured on a luminometer. Such method can be used to accurately measure as few as 5 cells per sample (or lower).

The foregoing, and other features and advantages of the present invention, will become more apparent from the following description and accompanying drawing.

Brief Description of the Drawings

Figure 1 shows a comparison of lysing methods on amount of light produced and detected.

Figure 2 shows the results varying the amount of luminescence reagent has on amount of light produced and detected.

Figure 3 shows the results varying sample-lysing agent incubation time has on light produced and detected.

Figures 4 and 5 show the results varying the amount of lysing agent has on amount of light produced and detected.

Figure 6 shows the kinetics of the light reaction for varying amounts of bacteria.

Figure 7 shows correlation of amounts of yeast to amount of light produced and detected.

Figure 8 shows correlation of amount of human white blood cells to amount of light produced and detected.

According to the present invention the luminescence flash response is obtained on polycarbonate membranes (such as Nucleopore® membranes available from Sargent-Welch, Chicago, Illinois) for concentrating microorganisms. Membranes having diameters of either 13 mm or 25 mm are usually used with 12 mm × 55 mm polystyrene tubes.

In use, the sample to be tested for unicellular organisms is filtered directly on conventional filtering equipment if it is in liquid form, or if the sample is in solid form, the cells can be conventionally extracted with such things as buffer solutions or water. It should be noted that while descriptions herein are made in terms of cells, actual measurements would be in terms of colony forming units which are roughly equivalent.

Filtering equipment typically used includes : (a) 13 mm Swinney filter luer-locked to a 1 cc to 50 cc sterile disposable syringe ; (b) 200 ml Gelman funnel filter (calibrated in 25 ml) seated on 250 ml vacuum flask with filtration achieved by hand vacuum pump (or in-line vacuum).

Sufficient lysing reagent should be used to lyse all of the cells in the sample being tested. While most conventional biological cell lysing materials such as boiling Tris (tris (hydroxy methyl) aminomethane hydrochloride) can be used to lyse the cells and release the cellular ATP, quaternary ammonium salt based PICOEX® B (Packard Instrument Co., Downers Grove, Illinois) is preferably used with the present

0 101 398

invention. Approximately 100 microliters of PICOEX B with either the 13 mm or 25 mm diameter filter membrane has been found to be optimal in most instances. If the boiling Tris method is used, the sample is boiled at a temperature above 100 °C for in excess of five minutes to achieve complete lysing of the sample.

Once the cells have been lysed and the ATP released, the luminescent reagent is added. While any luminescent reagent which produces light in the presence of ATP may be used, firefly luciferase-luciferin based PICOZYME™ F (Packard Instrument Co.) is preferably used with the present invention. Here again, sufficient luminescent reagent is added to react with all of ATP released from the cells collected on the filter membrane. For example, about 160 microliters of PICOZYME F has been found to be optimal for a 13 mm or 25 mm diameter filter membrane.

The polycarbonate filter membranes containing the concentrated cells are generally inserted in a test tube, for example, a polystyrene tube 12 mm in diameter and 55 mm in length has been found to be particularly suitable. It has also been found that even though the filter membrane may curl along the walls of the test tube or fold upon itself, accurate measurements of light on the luminometer are still obtained when the reagents are applied sequentially as described herein.

While any luminometer may be used with the present invention, the PICOLITE™ Model 6112 (Packard Instrument Co.) has been found to be particularly suitable. Utilizing the PICOZYME F with the PICOLITE luminometer, one photon of light is theoretically produced for each ATP molecule reacted.

Example 1

A 13 mm diameter polycarbonate filter membrane is placed inside a Swinney filter holder. The whole unit is then sterilized by autoclaving. A luer-tip sterile syringe (1 cc to 50 cc volume) is connected to the Swinney filter holder. The plunger is removed from the syringe and the desired volume of sample (0.1 cc to 50 cc) is poured into the syringe. The plunger is replaced and with a slow, even pressure the solution is forced through the Swinney filter to concentrate the cells on the membrane. The membrane is retrieved and placed into a 12 mm × 55 mm tube with the cell side concaved. A specific volume of each dilution of *E. coli* was used to give a fixed final cell count on the membrane of $8.4 \times 10^4$. One hundred microliters of the lysing agent PICOEX B were added to the membranes. After a five minute incubation period at room temperature, 160 microliters of PICOZYME F were added to the membrane. Immediately after the PICOZYME F is added, the tube is placed into the detecting chamber of the luminometer with the membrane in it and light response is measured for 30 seconds. The results, as an average of three assays, are shown in Table I.

Table I

| Volume of Sample Containing $8.4 \times 10^4$ Cell | Light Response (Counts/30 Seconds) |
|---|---|
| 40 μl | 250,511 |
| 3 ml | 254,975 |
| 10 ml | 255,043 |
| 50 ml | 240,514 |

Example 2

A 25 mm diameter polycarbonate filter membrane is placed on a support screen of a polysulfone twist-lock filter funnel. The top part of the filter funnel is twist-locked onto the base. The whole unit is autoclaved and placed on a vacuum flask. The desired volume of sample (up to 200 ml) is poured into the funnel. A vacuum is pulled such that the sample is filtered through the membrane. The filtrate is collected in the flask and the cells retained on the membrane. The top part of the filter is disassembled from the bottom of the filter, the membrane retrieved and placed into a 12 mm × 55 mm polystyrene tube as in Example 1, with cell side concaved. The lysing and luminescent reagents are added as in Example 1. As in Example 1, the specific volume of each dilution of *E. coli* filtered was such as to give a known final cell count on the membrane of approximately $8.4 \times 10^4$. The results are shown in Table II.

Table II

| Volume of Sample Containing $8.4 \times 10^4$ Cell | Light Response (Counts/30 Seconds) |
|---|---|
| 40 μl | 298,688 |
| 25 ml | 277,301 |
| 50 ml | 346,660 |
| 100 ml | 218,429 |
| 200 ml | 235,270 |

3

**0 101 398**

The results in Tables I and II demonstrate the applicability of concentrating bacteria from different sample volumes and with different types of filtering apparatus. The correlation of variation of light response obtained from all the data of these Tables is approximately 16 %.

### Example 3

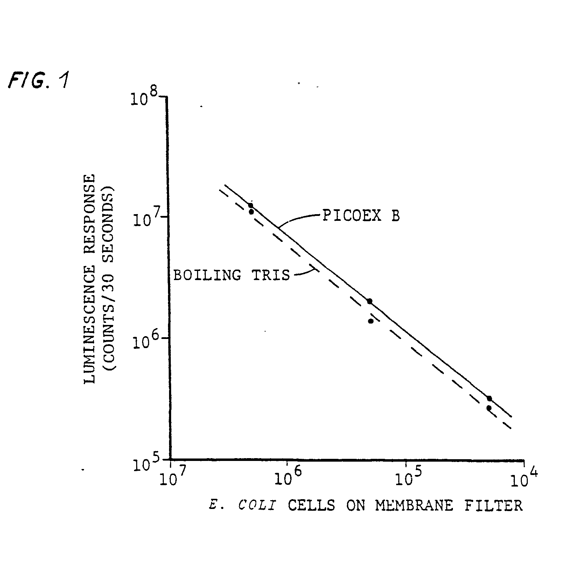

40 microliters of a sample solution containing $2 \times 10^4$ to $2 \times 10^6$ E. coli cells were filtered through a 13 mm diameter polycarbonate membrane. The cells were lysed with 100 µl of PICOEX B and the released ATP reacted with 160 µl of PICOZYME F. 0.5 ml of a sample solution containing $2.5 \times 10^6$ to $2.5 \times 10^8$ E. coli cells were mixed with 4.5 ml of a Tris-EDTA (ethylene diamine tetra-acetic acid) containing solution and boiled at 100 °C for at least 5 minutes. 40 microliters of this mixture were placed on a 13 mm diameter polycarbonate membrane and reacted on the membrane with 160 µl of PICOZYME F. A comparison of a 30 second count on the luminometer taken from both samples is shown in Fig. 1.

### Example 4

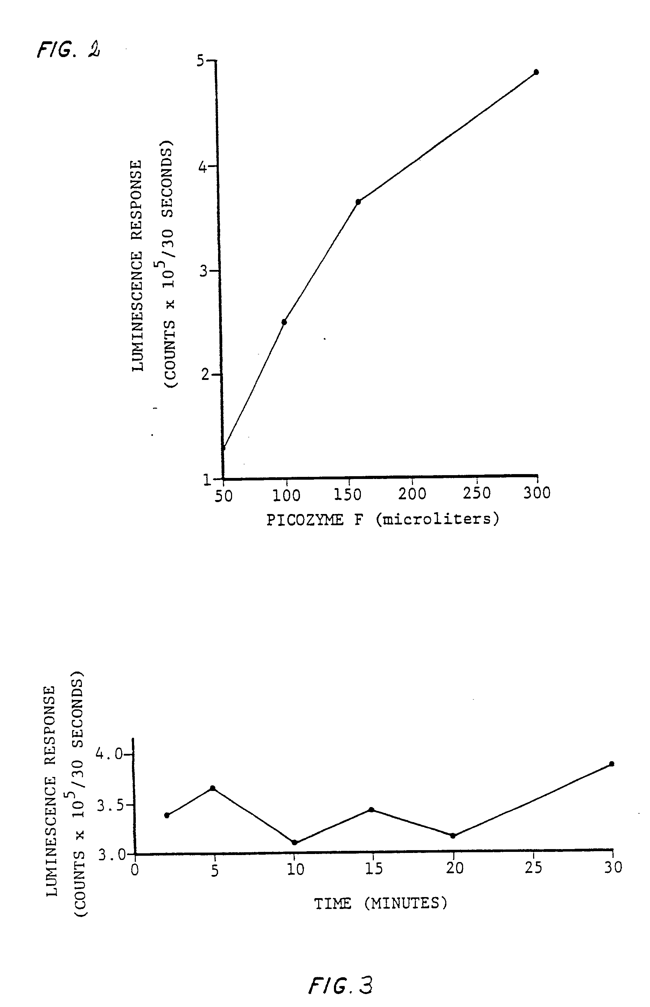

For an $8.4 \times 10^4$ E. coli containing sample varying amounts of PICOZYME F were added to the sample to optimize light response. The results of this experiment are shown in Fig. 2.

### Example 5

For an $8.4 \times 10^4$ E. coli containing sample, the PICOEX B lysing agent was allowed to incubate with the filter membrane for varying amounts of time. The results are shown in Fig. 3.

### Example 6

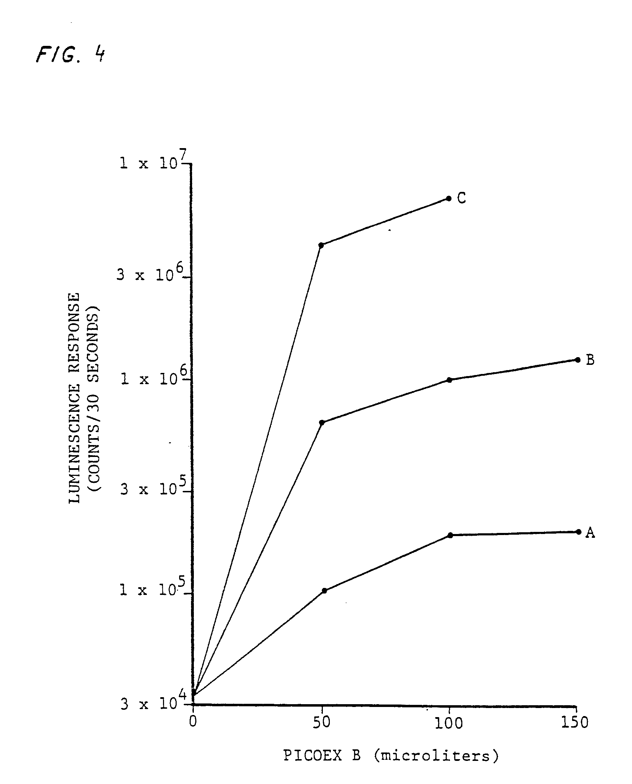

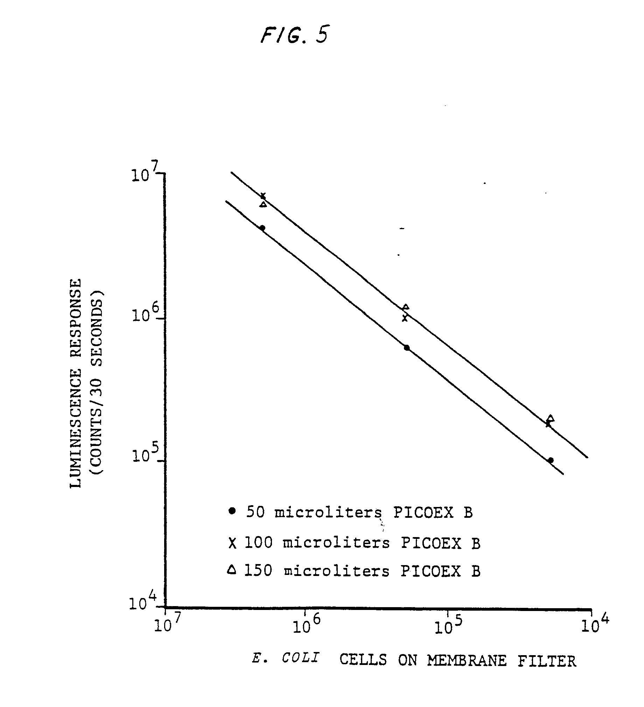

For three levels of E. coli containing samples (A = $2 \times 10^4$ ; B = $2 \times 10^5$ ; and C = $2 \times 10^6$), varying amounts of PICOEX B were added to the filter membrane. The results are shown in Figs. 4 and 5.

### Example 7

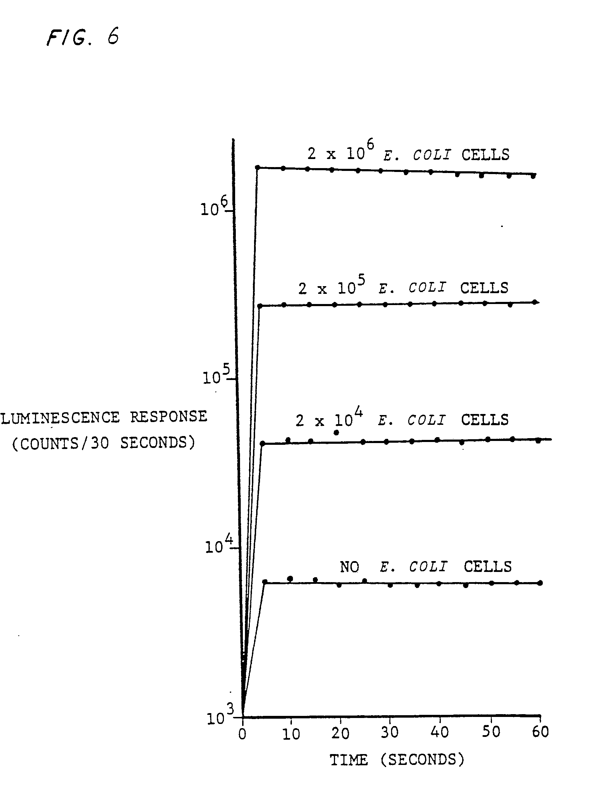

With varying amounts of E. coli on the membrane, the time for response after addition of the PICOZYME F was noted. The results are shown in Fig. 6.

Although not limiting, the results of the above Examples appear to indicate that approximately 100 microliters of lysing agent (PICOEX B) incubating with the cells for about 5 to 30 minutes followed by treating with approximately 160 microliters of luminescent reagent (PICOZYME F) and measuring light response for 5 to 30 seconds on the luminometer is optimum.

Similar techniques can be adapted to detect yeast and white blood cells.

### Example 8

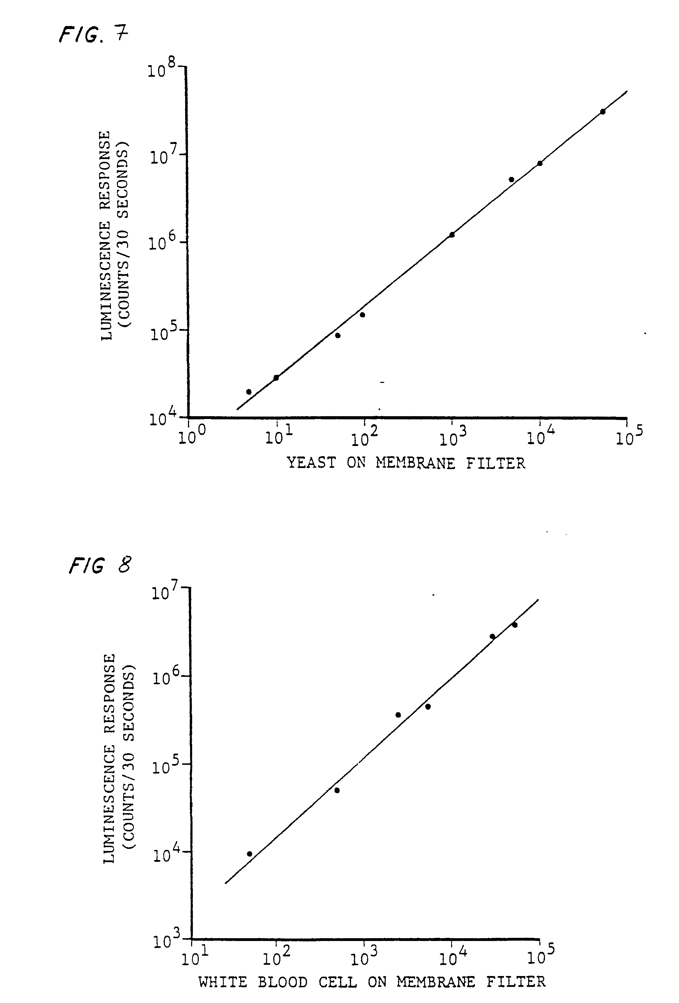

Ten microliters of solutions containing varying amounts of yeast (Saccharomyces cerevisiae) cells (5 to 50 000) were added to 13 mm diameter polycarbonate membranes and after a 5 minutes treatment with 10 µl of PICOEX B, the membrane was flashed with 50 µl of PICOZYME F. The results are shown in Fig. 7.

### Example 9

Increasing volumes of solutions containing a fixed amount of yeast (1 ml of 5 000 yeast cells/ml to 200 ml of 25 yeast cells/ml) are filtered through 13 mm or 25 mm diameter polycarbonate membrane. Cells retained on the membrane are lysed 5 minutes with 100 µl PICOEX B and assayed with 160 microliters PICOZYME F to give similar light response. Note Table III.

Table III

| Sample Volume | Light Response (Counts $\times 10^6$/30 sec.) |
|---|---|
| 1 ml* | 1.80 |
| 5ml* | 1.43 |
| 10 ml* | 0.92 |
| 50 ml** | 1.69 |
| 200 ml** | 1.33 |

\* Swinney filter, 13 mm diameter membrane

\*\* Gelman funnel filter, 25 mm diameter membrane

4

## Example 10

The total human white blood cell (WBC) content of a sample (separated from whole blood with Ficoll-Renographin gradient centrifugation) can be assayed on a model 6112 PICOLITE luminometer by flashing the 13 mm membrane-filtered cells with 50 microliters of PICOZYME F after 5 minutes lysing with 10 microliters of saponin based PICOEX™ S (Packard Instrument Co.). The linear detecting range is 50 to 50 000 viable cells per assay. Note Fig. 8.

## Example 11

Solutions with a known amount of WBC ($2.5 \times 10^3$ cells) were filtered through 13 mm or 25 mm polycarbonate membrane and assayed in the same manner as described in Example 9. Regardless of the method of concentration (Swinney filter holder or funnel filter) and the volume of the sample (1 ml to 200 ml), the same amount of light response is obtained when the cells collected on the membrane were flashed. Note Table IV.

### Table IV

| Sample Volume | Light Response (Counts $\times 10^6$/30 sec.) |
|---|---|
| 1 ml* | 0.35 |
| 5 ml* | 0.36 |
| 10 ml** | 0.33 |
| 50 ml** | 0.34 |
| 200 ml** | 0.42 |

\* Swinney filter, 13 mm diameter membrane
\*\* Gelman funnel filter, 25 mm diameter membrane.

As seen from the above, not only does the present invention provide a relatively simple and fast procedure to detect cells, but samples containing minute amounts of cells can be concentrated quickly for accurate measurement. For example, samples containing less than $1 \times 10^5$ cells per milliliter can be measured according to the present invention. Furthermore, entire samples containing less than 500 cells (E. coli) and even as few as about 10 cells (yeast) per sample can be detected with the process of the present invention. This procedure provides the additional benefit of a very « portable » assay method. While not intending to limit its utility, the present process provides a useful method for measuring bacteria in such things as drinking water, wine yeast content, organisms in cooling tower water, organisms in oil field flooding solutions, organisms in petroleum products such as jet fuels or lubricating oils, etc., all of which have certain microorganism level limits for efficient use.

## Claims

1. Method of measuring the concentration of ATP-containing unicellular organisms in a sample, comprising the steps of concentrating the organisms by passing the sample through a filter membrane impermeable to the organisms, rupture the organisms to release ATP from said organisms, treating with a luminescent reagent to react with released ATP, thereby producing a luminescence directly on the filter membrane and measuring the luminescent response with a luminometer characterized in that as said filter membrane a polycarbonate filter membrane is used and for rupturing the organisms, the filter membrane containing the concentrated organisms, is treated sequentially with a lysing agent.

2. The method of claim 1 characterized in that the sample contains at least 5 cells per sample.

## Patentansprüche

1. Verfahren zum Messen der Konzentration von ATP enthaltenden einzelligen Organismen in einer Probe, beinhaltend die Schritte des Konzentrierens der Organismen durch Hindurchleiten der Probe durch eine Filtermembran, die für die Organismen undurchlässig ist, des Aufbrechens der Organismen, um ATP aus den Organismen freizusetzen, des Behandelns mit einem lumineszierenden Reagenz, um es mit dem freigesetzten ATP reagieren zu lassen und dadurch eine Lumineszenz direkt auf der Filtermembran zu erzeugen, und des Messens der erzeugten Lumineszenz mit einem Luminometer, dadurch gekennzeichnet, daß als Fitermembran eine Polycarbonatfiltermembran benutzt wird und daß zum Aufbrechen der Organismen die Filtermembran, die die konzentrierten Organismen enthält, sequentiell mit einem Auflösemittel behandelt wird.

2. Verfahren nach Anspruch 1, dadurch gekennzeichnet, daß die Probe wenigstens 5 Zellen pro Probe enthält.

**Revendications**

1. Procédé en vue de mesurer la concentration d'organismes unicellulaires contenant du triphosphate d'adénosine dans un échantillon, ce procédé comprenant les étapes qui consistent à concentrer les organismes en faisant passer l'échantillon à travers une membrane filtrante imperméable aux organismes, provoquer la rupture des organismes afin d'en libérer le triphosphate d'adénosine, procéder à un traitement à l'aide d'un réactif luminescent en vue d'une réaction avec le triphosphate d'adénosine libéré, produisant ainsi une luminescence directement sur la membrane filtrante, puis mesurer la réponse luminescente à l'aide d'un luminomètre, caractérisé en ce que, comme membrane filtrante, on utilise une membrane filtrante en polycarbonate tandis que, pour la rupture des organismes, on soumet la membrane filtrante contenant les organismes concentrés à un traitement séquentiel à l'aide d'un agent de lyse.

2. Procédé selon la revendication 1, caractérisé en ce que l'échantillon contient au moins 5 cellules.

FIG. 1

FIG. 2

LUMINESCENCE RESPONSE
(COUNTS × $10^5$/30 SECONDS)

PICOZYME F (microliters)

LUMINESCENCE RESPONSE
(COUNTS × $10^5$/30 SECONDS)

TIME (MINUTES)

FIG. 3

*FIG. 4*

*FIG. 5*

LUMINESCENCE RESPONSE (COUNTS/30 SECONDS)

10⁷

10⁶

10⁵

10⁴

● 50 microliters PICOEX B

X 100 microliters PICOEX B

△ 150 microliters PICOEX B

10⁷    10⁶    10⁵    10⁴

*E. COLI* CELLS ON MEMBRANE FILTER

FIG. 6

FIG. 7

LUMINESCENCE RESPONSE (COUNTS/30 SECONDS)

YEAST ON MEMBRANE FILTER

FIG 8

LUMINESCENCE RESPONSE (COUNTS/30 SECONDS)

WHITE BLOOD CELL ON MEMBRANE FILTER